# EUROPEAN PATENT APPLICATION

(11) **EP 1 640 048 A1**
(43) Date of publication of application: **29.03.2006**
(21) Application number: 04731775.5
(22) Date of filing: 07.05.2004
(51) Int. Cl.: B01D 9/02

(54) **METHOD OF CRYSTALLIZING ORGANIC OLIGOMER, EPOXY RESIN COMPOSITION CONTAINING ORGANIC OLIGOMER OBTAINED BY THE METHOD AND EPOXY RESIN CURED MATERIAL**

(30) Priority: 09.05.2003 JP 2003132229
(71) Applicant: Tohto Kasei Co., Ltd., Tokyo 103 (JP)
(72) Inventor: NAKAMURA, Yoshiaki c/o Tohto Kasei Co., Ltd., 1-chome Chuo-ku, Tokyo 103-0002 (JP); NAKAMURA, Yukio c/o Laboratory, Tohto Kasei Co., L, Edogawa-ku, Tokyo 134-0084 (JP); YAJIMA, Hideaki c/o Kobe Factory,Tohto Kasei Co., Higashinada-ku kobe shi, Hyogo 658-0042 (JP); INAZUMI, Shojic/o Kobe Factory, Tohto Kasei Co.,, Higashinada-ku kobe shi, Hyogo 658-0042 (JP); ASAKAGE, Hideyasu c/o Laboratory, Tohto Kasei Co.,, Toky o 134-0084 (JP)
(74) Representative: Albrecht, Thomas
(86) International application number: PCT/JP2004/006520
(87) International publication number: WO 2004/098745

(57) **Abstract**

For the purpose to provide a method to crystallize organic oligomer of low crystallizing speed without using a specific apparatus and without wasting huge energy, and to provide epoxy resin composition suited as the sealing material for semi-conductor containing the organic oligomer obtained by said method following method is found out. That is, the method for crystallization of organic oligomers comprising, after fluidizing powdered or granulated seed crystals of organic oligomer in an apparatus with powder stirring mechanism, pouring liquid of same crystallizable organic oligomer continuously or by batch into said apparatus so that to mix and disperse, then growing up crystals of the organic oligomer in said apparatus.

## Description

### FIELD OF THE INVENTION

The present invention relates to the method for preparation of a novel method for crystallization of organic oligomer in the field of chemical industry, food industry and petroleum industry, especially relates to provide the method for preparation of a new method for crystallization of crystallizable epoxy resin oligomer.

### DESCRIPTION OF THE PRIOR ART

In various industries, a unit operation of crystallization is applied widely. Typically, said unit operation can be divided to two types, that is, one is crystallization from poor solvent and another one is crystallization from molten state. However, the crystallization from poor solvent needs many processes such as separation of crystal from mother liquor, drying process of crystal or recovering process of solvent from mother liquor, and can not be said as a profitable method. While, the crystallization from molten state has a limitation from the view point of equipment, and specifically, the methods to extrude as strands by an extruder, to cool on a belt flaker, to knead and cool in a kneader or to cool in a flat container are known. However, in the case when melt viscosity of organic oligomer is low and physical intensity at molten state is insufficient, the extruder can not be used. Further, in the case of the belt flaker, it is necessary to complete the crystallization in several minutes (about 2-5 minutes) and to solidify so as to be removed from the belt. Otherwise, the method to cool and crystallize in separated container can be mentioned.

As mentioned above, in the case which can use a kneader or a belt flaker, industrially continuous production is possible, on the contrary, in the case of crystallization by cooling in a separated container, it takes long time to the crystallization. During the process, the space to store the container is necessary, the operation to remove the product from the container and the shape of product removed from the container is coarse and has sharp angle and is dangerous for handling. Further, it is necessary to make the size of crystal finer, and has a possibility to contaminate foreign substances during the granulating process, and is not suited as the effective industrial production.

Regarding the crystallization of epoxy resin as the crystalline organic oligomer, in the Patent Document 1, the processing by molding characterized crystallizing and soldificating said resin by adding crystalline nucleus (curing agent or filling agent) to liquid epoxy resin in supercooling state at room temperature is disclosed. Since this method is characterized to add foreign substances as crystalline nucleus, this method can not be applied to the case which handles pure substance.

In the Patent Document 2, the following method for crystallization is disclosed. That is, to the epoxy resin of diglycidylether of 3,3',5,5'-tetramethyl-4,4'-dihydroxydiphenylmethane in the state of molten liquid, fine particles of said epoxy resin crystal are added and crystallized by stirred under the state maintaining liquid state at the temperature lower at least 20°C than a melting point of the epoxy resin. By adding seed crystals, the time for crystallization can be shortened, however, by this method, an extruder or a belt flaker can not be used and is necessary to be cooled and crystallized in a separated container, and the realization of industrial production is difficult.

In the Patent Document 3, the method for preparation of granular product of epoxy resin comprising, growing crystalline nucleus maintaining the crystallizable epoxy resin which is in supercooling or molten state at the temperature between 40°C or mores and lower than melting point then granulating, is disclosed. However, although this method is the method to form strands by a twin screw extruder and to granulate by a pelletizer, it is not possible to form strands in the case when the crystallizable epoxy resin of lower viscosity is used, further the time necessary for the crystallization is very long. In the Patent Document 4, the crystalline solid epoxy resin is obtained by cooling the molten state resin of diglycidylether of 2,7-naphtalenediol to the temperature lower than 30°C in a kneader so as to crystallize and by shearing it. However, since this method requires very huge stirring power, and is characterized as a batch production, this method is an ineffective method and is not suited for the industrial production.

In the Patent Document 5, the following method is disclosed. That is, after mixed and solubilized the crystallizable epoxy resin and the non crystallizable epoxy resin at the temperature more than the melting point of the former and cooling down by the cooling speed lower than 5°C/min, under stirring speed of lower than stirring Reinold's number 20. However, this method can not be applied to the case of crystallizable epoxy resin whose crystallization speed is slow.

As mentioned above, in the conventional arts, the crystallization method suited for the industrial scale production of organic oligomers whose crystallization speed is slow and whose viscosity is low is not proposed yet.
Patent Document 1 : JP Patent Laid-Open Publication 6-198644
Patent Document 2 : JP Patent Laid-Open Publication 7-179564
Patent Document 3 : JP Patent Laid-Open Publication 9-324029
Patent Document 4 : JP Patent Laid-Open Publication 2000-119369
Patent Document 5 : JP Patent Laid-Open Publication 2001-114983

### DISCLOSURE OF THE INVENTION

The inventors of the present invention have investigated variously about the method for crystallization suited for the industrial scale production of organic oligomers whose crystallization speed is slow and whose viscosity is low and accomplished the present invention. The object of the present invention is to provide a method for crystallization which promises an effectivity and profitability for the industrial production even if the crystallizable organic oligomer whose crystallizing speed is slow and which can not form strand because of low melt viscosity.

The important point of the present invention is a method for crystallization of organic oligomers comprising, after fluidizing powdered or granulated seed crystals of an organic oligomer in an apparatus with powder stirring mechanism, pouring liquid of same crystallizable organic oligomer continuously or by batch into said apparatus so that to mix and disperse, then growing up crystals of the organic oligomer in said apparatus.

That is, in the present invention, previously crystallized organic oligomer is crushed to powder or granule and use said crushed powder or granule of the organic oligomer as the seed crystals, and fluidizing said seed crystals by stirring in an apparatus with powder stirring mechanism. In this state, same crystallizable organic oligomer of liquid state is poured into said apparatus by continuous operation or by batch operation so that mix and to disperse and to grow up crystals in said apparatus.

And according to one embodiment of this method, it is desirable that the crystallization temperature is lower at least 10°C than the melting point of said organic oligomer and is within the temperature range of from 200°C to 0°C. Further, it is desirable to remove the heat of crystallization by a cooler attached to the apparatus with powder stirring mechanism or to introduce previously cooled inert gas into the apparatus with powder stirring mechanism so as to exchange the heat by contacting with organic oligomer particles in said apparatus.

Further, in the present invention, it is desirable to produce organic oligomer crystals continuously by picking out a part of or whole organic oligomer crystals from the apparatus with powder stirring mechanism, and provide powdered or granulated organic oligomer crystals again continuously or by batch operation to the apparatus with powder stirring mechanism, desirably passing through a pulverizer, as seed crystals.

In the present invention, it is further desirable that the organic oligomer is crystallizable resin low moleculer polymer, and in particular, it is furthermore desirable that the crystallizable resin low moleculer polymer is crystallizable epoxy resin low molecular weight polymer. And, desirably the crystallized ratio of the organic oligomer crystal is 65% or more.

### BRIEF DESCRIPTION OF THE DRAWINGS

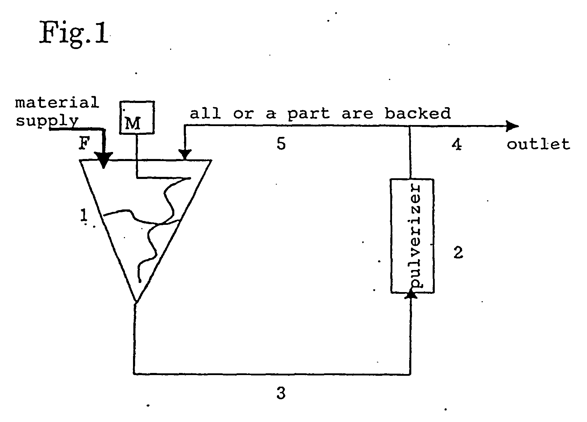

Fig.1 shows the flow of the process of continuous crystallization of organic oligomers, and 1 is an apparatus with powder stirring mechanism, 2 is a pulverizer, 3 is an inlet route, 4 is an outlet route, 5 is a circulation route, F is a molten oligomer feed line and M is a screw.

### DESCRIPTION OF THE PREFERRED EMBODYMENT

The present invention will be illustrated more in detail according to the following description.
The term of crystallizable organic oligomer indicates a crystallizable resinous state low molecular weight polymer whose molecular weight is from 200 to 2000. As the examples of said crystallizable organic oligomer, aromatic polycarbonate, aromatic polyester, poly lactic acid resins or aromatic epoxy resin can be mentioned, however, not intending to be limited to them, and any organic oligomer with crystallinity can be applied.

Further, at the pouring of liquid state crystallizable organic oligomer, both molten state liquid of the temperature higher than melting point and supercooled liquid of the temperature lower than melting point can be used, however, the temperature is desirably within the range of 30°C higher than the melting point and 20°C lower than the melting point, because the efficiency for the generation of crystals is high. When the temperature is more than 30°C higher than the melting point, the cooling efficiency in the apparatus with powder stirring mechanism becomes low, while, the temperature is more than 30°C lower than the melting point, the efficiency of generation of crystals becomes low.

As the apparatus with powder stirring mechanism of the present invention, any type of a powder mixing tank with a stirring impeller or a fluidized bed mixing tank which can introduce fluid gas from bottom can be used. As the powder mixing tank with a stirring impeller, a horizontal mixer with ribbon shape stirring propeller, a vertical V type mixer (Nauter type, Helical ribbon type) can be mentioned, in particular, a type which inner screw rotates and revolves can be desirably used. Further, for the purpose to remove the heat of crystallization, it is desirable to install a heat transferring device, such as jacket. It is possible to pass coolant such as water in the jacket, however, in the case that the heat transferring area is too small, it is possible to blow cooled nitrogen gas, cooled carbon dioxide gas or cooled air directly to the mixer and to cool the contents. As the pulverizer, batch type crushing method or continuous crushing method can be used. As the specific example of the crushing machine, jaw crusher, gyratory crusher, hammer crusher, roll crusher, ball mill, edge-runner, hammer mill, pin mill, cutter mill, feather mill, vibration rod mill, cascade mill, turbo mill, pot mill, compound mill, radial mill, tower mill, circular vibration mill, disk mill, high swing ball mill, centrifuge ball mill, atomizer, pulverizer, super micron mill, jet mill or colloid mill can be mentioned.

The important point in the method for crystallization of the present invention is to drop crystallizable organic oligomer of liquid state into seed crystals which is powdered or granulated organic oligomer and to adhere the seed crystals on the surface of liquid drop and to disperse the liquid drop to the surface of which seed crystals are adhered into powdered or granulated organic oligomer. Further, at the picking out the organic oligomer crystals continuously as the product, it is desirable that a part of it is provided again continuously or by batch operation to the apparatus with powder stirring mechanism as seed crystal and carry out the crystallization of crystallizable organic oligomer continuously by adjusting the amount of liquid crystallizable organic oligomer to be supplied, the residence amount in the apparatus with powder stirring mechanism and temperature. At the stirring and cooling process in the powder mixer, the crystallization of said liquid drop is proceeded rapidly by many seed crystals, by impact and shearing caused by stirring and friction, and new particles (crystals) are generated by friction between particles. In the case when the generation of organic oligomer crystals is small, it can be improved by picking out a part of product from said apparatus with powder stirring mechanism and put back it to the apparatus after crushed. The term of powdered or granulated (hereinafter shortened to fine particles) means to contain at least 10%, desirably 20% more desirably 30% of 100 mesh passed particles. In the present invention, it is desirable to make the size of liquid drop which is provided to the stirring mixer smaller, or to drop from porous substance so as to disperse smoothly in fine particles desirable. In the present invention, when the numbers of fine particles oligomer to be seed crystals become lacking, the size of particles in the stirring mixer becomes larger gradually. Although the size of particle in the stirring mixer depends on the specie of organic oligomer, generally forms random shape particles of approximately 1mm to 50mm, and since a part of particles are crushed by collision of particles themselves, the shape of particles is apparently round without sharp edges. Therefore, the picking out operation and handling operation of the product becomes easy.

The present invention can be applied not only to the oligomer whose crystallization speed is fast, but also to the oligomer whose crystallization speed is slow. In the present invention, the desirable embodiment is to pick out the corresponding amount to the provided amount to the apparatus with powder stirring mechanism as the product continuously or by batch operation. Further, the most suitable residence (crystallization) time of various crystallizable organic oligomers can be decided by adjusting the residing amount in the apparatus with powder stirring mechanism, for example, by adjusting the variation of the residing amount in the apparatus with powder stirring mechanism, supplying amount of liquid organic oligomer and the amount the picking out amount of the crystal product. In the present invention, the long crystallization (residing) time is not required, however, the desirable crystallization time is from 5 minutes to 10 hours, more desirably is from 10 minutes to 5 hours and furthermore desirably is from 30 minutes to 3 hours.

The desirable temperature at the crystallizing is the temperature which is 10°C or more under (lower) than the melting point of said organic oligomer (melting peak temperature of differential scanning calorimeter (DSC) curve), more desirable temperature at the crystallizing is the temperature 20°C or more under than the melting point of said organic oligomer. By too lower temperature, the surface of fused liquid solidified by amorphous state (non-crystalline state) and does not adhere seed crystals. The desirable crystallization temperature is from 200°C to 0°C, more desirably is from 160°C to 10°C, furthermore desirably from 140°C to 10°C.

As the crystallizable epoxy resin oligomer which can be used in the present invention, any epoxy resin with crystallinity can be used and not limited by the chemical structure, and following compounds can be mentioned as the example.
Diglycidylether compound of 3,3',5,5'-tetramethyl-4,4'-dihydroxydiphenyl methane,
diglycidylether compound of 3,3'-dimetyl-4,4'-dihydroxydiphenylmethane,
diglycidylether compound of 2,2'-3,3'-5,5'-hexamethyl-4,4'-dihydroxy diphenylmethane,
diglycidylether compound of 1,4-bis(4-hydroxycumyl)benzene,
diglycidylether compound of 2,2'-dimethyl-5,5'-ditertiarybutyl-4,4'-dihydroxydiphenylmethane,
diglycidylether compound of 1,4-bis (3,5dimethyl-4-hydroxycumyl)benzene,
diglycidylether compound of 4,4'-dihydroxydiphenylether,
diglycidylether compound of 4,4'-dihydroxy diphenylsulfide,
diglycidylether compound of 2,2'-dimethyl-5,5'-ditertiarybutyl-4,4' -dihydroxydiphenylsulfide,
diglycidylether compound of 2,5-ditertiarybutylhydroquinone,
diglycidylether compound of 4,4'-dihydroxy biphenyl,
diglycidylether compound of 3,3',5-5'-tetramethyl-biphenol,
polyglycidylether compound of biphenylalalkylphenol,
polyglycidylether compound of phenolalalkylnapthol,
diglycidylether of tetrabrombisphenol A,
epoxy compound with mesogen structure or
stilben epoxy resins can be mentioned.

The crystallization ratio of the organic oligomers immediately after the crystallization obtained by the method for crystallization of the present invention is desirably to be 65% or more, more desirably to be 75-95%. When the crystallization ratio is smaller than 65%, the problem of blocking can be generated and is inferior in preserving stability and is inferior of the function as seed crystals. On the contrary, when the crystallization ratio exceeds 95%, the crystallization (preservation) time becomes long and is not advantageous from the productive efficiency. The crystallization ratio of the present invention can be measured from the colorimetric ratio between fusing calorie of obtained crystal of organic oligomers measured by DSC and the fused calorie of standard specimen whose purity of crystalline component (component containing epoxy resin at both ends) is enhanced to more than 99% (area % measured by GPC) by re-crystallization using poor solvent to the crystal. As the poor solvent, the compound selected from the group consisting of alcohols such as methanol, ketones such as methylethylketone or methylisobutylketone, toluene or xylene can be mentioned.

The crystallizable epoxy resin composition of the present invention can be used together with other kinds of epoxy resin in the limitation not spoiling the characteristics of the crystallizable epoxy resin of the present invention. As the epoxy resin which can be used together with, any kinds of oligomer or polymer possessing two or more epoxy groups in one molecule can be mentioned. For example, orthocresolnovolac epoxy resin, phenolnovolac epoxy resin, dicyclopentadiene modified phenol epoxy resin, naphthol epoxy resin, triphenolmethane epoxy resin, phenolalalkyl (possessing phenylene structure or diphenylene structure) epoxy resin can be mentioned, and these resins can be used alone or together with.

The crystallizable epoxy resin composition of the present invention is preferably the crystallizable epoxy resin composition for semi-conductor sealing material, and can contain said crystallizable epoxy resin, other epoxy resin, curing agent such as phenol resin, curing accelerating agent such as tertiary amine, imidazole or phosphine, inorganic filler composed of inorganic powder such as silica or alumina, coupling agent such as silane coupling agent, releasing agent such as carnauba wax, stearic acid or coloring agent such as carbon black.

As the phenol resin which can be used in the crystallizable epoxy resin of the present invention, any kinds of oligomer or polymer possessing two or more phenolic hydroxy groups in one molecule can be mentioned, specifically, phenolovolac resin, phenolalalkyl (possessing phenylene structure or diphenylene structure) epoxy resin, naphtholalalkyl (possessing phenylene structure or diphenylene structure) resin, terpene modified phenol resin, dicyclopentadiene modified phenol resin or naphthol resin can be mentioned and these resins can be used alone or together with. Molecular weight, softening point or hydroxyl equivalent of these resins are not specifically restricted, however, phenol resin having relatively lower melt viscosity which is solid at room temperature and whose softening point is 110°C or less is desirable. When the softening point exceeds 110°C, the viscosity of the epoxy resin composition becomes high and not desirable.

The inorganic filler which can be used in the present invention is not restricted, and the inorganic filler which is ordinary used can be used. For example, fused and crushed silica powder, fused spherical silica powder, crystalline silica powder, secondary flocculated silica powder, alumina, titanium white and aluminum hydroxide can be mentioned, and among these compounds, fused spherical silica powder is preferably used. The shape of these fillers is desirably to be as much as spherical, and the filling amount can be improved by mixing use of different particle sizes. The desirable blending amount of the inorganic filler used in the present invention is 70-95 wt% to molding material, and more desirable amount of it is 75-93 wt%. When the filling amount of the inorganic filler is less than 75 wt%, moisture absorption amount increases, and when is used as the semi-conductor sealing material, cracks are easily generated in semiconductor device at the process of soldering reflow treatment. On the contrary, when the filling amount of the inorganic filler exceeds 95wt%, the fluidity of the molding material at the molding process deteriorates and the problems of non-filled, chip shift or die pad shift of the semi-conductor unit becomes easily generated and is not desirable.

As the curing accelerating agent which can be used in the present invention, for example, amidine compounds such as 1,8-diazabicyclo (5,4,0)undecene-7, organicphosphorous compounds such as triphenyl phosphine or tetraphenylphosphonium · tetraphenylborate or imidazole compounds such as 2-methylimidazole can be mentioned, however, not intending to be limited to them. These curing accelerating agent can be used by alone or together with.

The crystallizable epoxy resin composition used in the present invention can contain additives, for example, flame retardant agent such as brominated epoxy resin, antimony oxide, phosphorous compound, inorganic ion exchanger such as bismuth oxide hydrate, coupling agent such as γ -glycidoxypropyltrimethoxysilane, coloring agent such as carbon black or red iron oxide, releasing agent such as carnauba wax or polyethylene wax, low stress agent such as silicone oil or silicone rubber or antioxidant, when need is arisen. As the specific sealing method of electric parts such as semi-conductor unit by solidified composition containing organic oligomer crystals, the molding method such as transfer mold, compression mold, injection mold can be used.

The process of continuous crystallization of the present invention will be more specifically illustrated according to Fig 1.
Fig.1 shows the process flow of the continuous crystallization of the present invention, and the apparatus with powder stirring mechanism 1 has a feed line for material supply F and a stirrer M. A crushing machine 2 has an inlet route 3, an outlet route 4 and a circulation route 5. In this kind of apparatus, liquid state organic oligomer is supplied to the apparatus with powder stirring mechanism 1 through F. In the apparatus with powder stirring mechanism 1, fluidize fine particles of seed crystals which is previously introduced by screw M and generate new crystals (random shape particles) by dropping liquid organic oligomer. Crystals grown to appropriate size are introduced into the crushing machine 2 through the inlet route 3. In the crushing machine 2, the introduced crystals are crushed to crystals having the desired particle size (fine particles). Said fine particles are picked out from the outlet route 4, and all or a part of the fine particles are backed to the apparatus with powder stirring mechanism 1 through the circulation route 5.

### EXAMPLES

The crystallization method of organic oligomer of the present invention will be illustrated more in detail according to the Example and Comparative Examples, however, not intending to be limited to them.

Melting point (temperature of main melting peak) and the ratio of crystallization is measured by a differential scanning calorimeter (DSC) under the condition of approximately 10mg amount of specimen, 10°C/min temperature elevating speed and in nitrogen atmosphere of 30mL/min. The crystallization ratio is measured from the colorimetric ratio (endothermic colorimetric ratio) between fusing calorie of specimens obtained in each Examples and the fused calorie of standard specimen whose purity of crystalline component (component containing diglycidyl ether which has zero degree of polymerization) is enhanced to more than 99% (area % measured by GPC) by re-crystallization using poor solvent to each obtained specimen. As the poor solvents used to the specimens obtained from Examples 1-3, methanol, methylisobutylketone and methylethylketone are respectively used. In cases of Comparative Example 1 and Example 4, methanol is used.

### Example 1

In a 360L Nauter type mixer with jacket, 100kg of fine particles of 3,3',5,5'-tetramethyl-4,4'-dihydroxydiphenylmethane diglycidylether of 73°C melting point, which was previously crystallized sufficiently by spontaneous cooling and crushed, was contained and fluidized by screw and the inner temperature was cooled down to 10°C by passing coolant through the jacket. Then 84kg of fused said resin liquid of 88°C was dropped on fluidized fine particles under constant stirring by 1 hour. During the dropping process, the inner temperature of the mixer elevated to 26-30°C. Crystals were dispersed as the random shape particles without sticking to the screw and the wall of mixer. The melting point of the crystals picked out after 2 hours from the dropping was 73°C, and new endothermic peak (2^{nd} peak) was observed at approximetely 60°C. Further, the crystallized ratio measured by DSC was 78%.

### Comparative Example 1

To the fused liquid of 3,3',5,5'-tetramethyl-4,4'-dihydroxydiphenyl methanediglycidylether of 75 °C , 0.5wt% of fine particles of 3,3',5,5'-tetramethyl-4,4'-dihydroxydiphenylmethanediglycidylether whose melting point is 73°C, which was previously crystallized and crushed, was added and mixed, then poured into a can of 20L capacity and crystallized by spontaneous cooling. Contents were solidified after 24 hours, and the crystallized ratio was 63% and 78% after 72 hours.

### Example 2

150g of fine particles of 3,3',5,5'-tetramethyl-4,4'-dihydroxybiphenol diglycidylether whose melting point is 105 °C , which was previously crystallized and crushed, was previously contained into a cylindrical separable flask of 500mL capacity with Herical ribbon impeller, and said cylindrical flask was dipped in water bath so that the inner temperature was cooled down to 20 °C . Then, 150g of previously fused 3,3',5,5'-tetramethyl-4,4'-dihydroxybiphenoldiglycidylether at 120 °C was dropped on the fine particles under constant stirring by 1 hour. During dropping process, the inner temperature of the separable flask elevated to 35°C, and the crystals were dispersed as random shape particles of 2-5mm size without sticking to the stirring impeller and to the inner wall of the flask. The melting point of the crystals picked out after 1 hour from the dropping was 105°C, and new endothermic peak (2^{nd} peak) was observed at approximetely 86°C. Further, the crystallized ratio measured by DSC was 85%.

### Example 3

The apparatus used in Example 2 was used. 150g of fine particles of 2,5-ditertiarybutylhydroquinonediglycidylether whose melting point is 141 °C , which was previously crystallized and crushed, was previously contained and cooled down by water bath. Then, 150g of previously fused diglycidylether of 2,5-ditertiarybutylhydroquinone at 160°Cwas dropped on the fine particles under constant stirring by 1 hour. During dropping process, the inner temperature of the separable flask elevated to 45°C, and the crystals were dispersed as random shape particles of 2-5mm size without sticking to the stirring impeller and to the inner wall of the flask. The melting point of the crystals picked out after 15 minutes from the dropping was 141°C, and new endothermic peak (2^{nd} peak) was observed at approximetely117°C. Further, the crystallized ratio measured by DSC was 90%.

### Example 4

In a 360L Nauter type mixer with jacket, 100kg of fine particles of 3,3',5,5'-tetramethyl-4,4'-dihydroxydiphenylmethanediglycidylether of 73°C melting point, which was crystallized sufficiently and crushed, was contained and fluidized by screw and the inner temperature was cooled down to 10°C by passing coolant through the jacket. A part of said fine particles were provided to a Nauter mixer as seed crystals through a crusher and circulated continuously. Then molten liquid of said resin of 88°C was dropped on fluidized fine particles under constant stirring, and a part of fine particles of crystals discharged from the crusher is picked out continuously as the product. During said process, the inner temperature of the mixer elevated to 21-25°C. Crystals in the Nauter type mixer were dispersed as random shape particles of 1-3mm without sticking to the screw or the inner wall of the mixer. The melting point of this product is 73°C, and new endothermic peak (2^{nd} peak) was observed at approximetely 60°C. Further, the crystallized ratio measured by DSC was 73%.

### Examples 5-7 and Comparative Examples 2-4

Blending ratio of compositions containing crystallizable organic oligomer is shown in Table 1. Numeric values in the Table shows the weight parts. Crystallizable organic oligomer, phenol novolac curing agent and additives shown in Table 1 were blended and previously mixed, then kneaded by a kneader at 100°C. After the products were cooled, crushed and the epoxy resin compositions of the blending ratio of Examples 5-7 and Comparative Examples 2-4 were obtained.

**Table 1**

| | Ex. 5 | Ex. 6 | Ex. 7 | Compar. Ex. 2 | Compar. Ex. 3 | Compar. Ex. 4 |
|---|---|---|---|---|---|---|
| kind of C.O.O. * | A | B | C | D | E | F |
| amount of C.O.O.* | 124.6 | 124.6 | 120.0 | 124.6 | 124.6 | 120.0 |
| (1) | 67.4 | 67.4 | 72.0 | 67.4 | 67.4 | 72.0 |
| (2) | 8 | 8 | 8 | 8 | 8 | 8 |
| (3) | 4 | 4 | 4 | 4 | 4 | 4 |
| carbon black | 2 | 2 | 2 | 2 | 2 | 2 |
| calcium stearate | 2 | 2 | 2 | 2 | 2 | 2 |
| triphenyl phosphine | 2 | 2 | 2 | 2 | 2 | 2 |
| silane coupler | 3 | 3 | 3 | 3 | 3 | 3 |
| fused silica | 884 | 884 | 884 | 884 | 884 | 884 |

| | | | | | | |
|---|---|---|---|---|---|---|
| * crystallizable organic oligomer | | | | | | |

Crystallizable organic oligomers A-F and (1)-(3) are illustrated as follows:
Organic oligomer A: Crystallizable diglycidylether compound of 3,3',5,5' -tetramethyl-4,4'-dihydroxydiphenylmethane obtained in Example 1, whose melting point is 73°C (epoxy equivalent is 193g/eq).
Organic oligomer B: Crystallizable diglycidylether compound of 3,3',5-5' -tetramethyl-biphenol obtained in Example 2, whose melting point is 105°C (epoxy equivalent is 193g/eq).
Organic oligomer C: Crystallizable 2,5-ditertiarybutylhydroquinone diglycidylether obtained in Example 3, whose melting point is 141°C (epoxy equivalent is 175g/eq).
Organic oligomer D: Diglycidylether of 3,3',5,5'-tetramethyl-4,4'-dihydroxydiphenylmethane which is used as seed crystals in Example 1(epoxy equivalent is 193g/eq).
Organic oligomer E: Diglycidylether of 3,3',5,5'-tetramethyl-4,4'-dihydroxy biphenyl which is used as seed crystals in Example 2 (epoxy equivalent is 193g/eq).
Organic oligomer F: 2,5-ditertiarybutylhydroquinonediglycidylether which is used as seed crystals in Example 3 (epoxy equivalent is 175g/eq).
Phenol novolac resin (1): Product of Showa Hi-Polymer Co., Ltd., BRG-555, softening point is 65°C, hydroxyl equivalent is 105g/eq
Flame retardant agent (2): Product of Tohto Kasei Co., Ltd., YDB-360, brominated bisphenol A epoxy resin whose epoxy equivalent is 360g/eq.
Flame retardant promoter (3): Antimony oxide

### Examples 8-10 and Comparative Examples 5-7

Crystallizable epoxy resin compositions of Table 1 were cured and the characteristics are measured. Results are shown in Table 2. Measuring methods are illustrated as follows.
1. Glass transition temperature
Measured by thermomechanical analysis (TMA) of the molded product which is cured at 180°C for 8 hours.
2. Water absorption ratio
Disk shape molded product of 50mm diameter and 2mm thickness which is cured at 180°C for 8 hours is used as a test piece. The specimen is set in a pressure cooker tester of 121°C, 100%RH condition and weight change after 120 hours is measured.

**Table 2**

| | Ex. 8 | Ex. 9 | Ex. 10 | Compar. Ex. 5 | Compar. Ex. 6 | Compar. Ex. 7 |
|---|---|---|---|---|---|---|
| kind of C.O.O. * | A | B | C | D | E | F |
| glass transition temp.( °C) | 128 | 130 | 135 | 128 | 130 | 135 |
| water absorption ratio (%) | 0.54 | 0.63 | 0.52 | 0.54 | 0.63 | 0.52 |

| | | | | | | |
|---|---|---|---|---|---|---|
| * crystallizable organic oligomer | | | | | | |

Obviously understood from the results of Examples 1-3, by crystallizing method of the present invention, not only easy crystallizable organic oligomer but also slow crystallizable organic oligomer can be effectively crystallized in short time. Further, as clearly understood from the results of Table 2, the epoxy resin composition of the present invention indicates similar characteristics to epoxy resin used as the seed crystals, and can be preferably used as the sealing material for semi-conductor.

### INDUSTRIAL APPLICABILITY

The present invention can be effectively used as the industrial method, which can crystallize organic oligomer of slow crystallization speed in short time without wasting a lot of energy, and can pick out continuously as the easy handling random shape particles. Further, the crystallized organic oligomer obtained by the method of the present invention can be preferably applied as the sealing material for semi-conductor.

## Claims

1. A method for crystallization of organic oligomers comprising, after fluidizing powdered or granulated seed crystals of organic oligomer in an apparatus with powder stirring mechanism, pouring liquid of same crystallizable organic oligomer continuously or by batch into said apparatus so that to mix and disperse, then growing up crystals of the organic oligomer in said apparatus.

2. The method for crystallization of claim 1, wherein the temperature to generate crystals in said apparatus is lower at least 10°C than from the melting point of said organic oligomer and is within the temperature range of from 200°C to 0°C.

3. The method for crystallization of claim 1 or claim 2, wherein the removal of the heat of crystallization is carried out by means of a heat exchanger attached to the apparatus with powder stirring mechanism or by introduction of previously cooled inert gas into the apparatus with powder stirring mechanism.

4. The method for crystallization according to anyone of claims 1 to 3, wherein the organic oligomer crystals is produced continuously by picking out a part of or whole organic oligomer crystals from the apparatus with powder stirring mechanism, and providing said a part of or whole organic oligomer crystals again continuously or by batch operation to the apparatus with powder stirring mechanism passing through a crushing machine.

5. The method for crystallization according to anyone of claims 1 to 4, wherein the organic oligomer is crystallizable resinous low molecular weight polymer.

6. The method for crystallization according to anyone of claims 1 to 5, wherein the organic oligomer is crystallizable epoxy resin lower molecular weight polymer.

7. The method for crystallization according to anyone of claims 1 to 6, wherein the crystallized ratio of the organic oligomer crystals is 65% or more.

8. A crystallizable epoxy resin composition comprising, the crystallizable epoxy resin lower molecular weight polymer obtained by the method of claim 6 or claim 7 and an epoxy resin curing agent.

9. A crystallizable epoxy resin composition used as the sealing material for electronic parts comprising, the crystallizable epoxy resin lower molecular weight polymer obtained by the method of claim 6 or claim 7 and an epoxy resin curing agent.

10. A cured product obtained by curing the epoxy resin composition of claim 8 or claim 9.
